# EUROPEAN PATENT APPLICATION

(11) **EP 0 968 692 A1**
(43) Date of publication of application: **05.01.2000**
(21) Application number: 99600007.1
(22) Date of filing: 23.06.1999
(51) Int. Cl.: A61F 2/44, A61B 17/70, A61B 17/80

(54) **Plate-cylinder vertebral prosthesis system**

(30) Priority: 29.06.1998 GR 98010024
(71) Applicant: Kalaitzis, Christos, 54642 Thessaloniki (GR)
(72) Inventor: Kalaitzis, Christos, 54642 Thessaloniki (GR)

(57) **Abstract**

It is a telescopic cylinder(8,9)-plate (20) spinal prosthesis system for the replacement of one or more bodies of the spine, Lumbar, Thoracic or Cervical, including the disks, while it restores the height back to the normal in case of trauma injuries or other cause, and it secures and 'ties' in to four points with the upper and lower healthy body in to compression position, thus, preventing the substitute to be moved from its position due to shearing forces. This goal could be reached because it all acts, reacts and behaves as one solid unit.

It also ensures cohesion and continuity of the graft which fills and bridges the gap between the healthy vertebral bodies so that the best conditions of its incorporation and secondary spine fusion will occur. Normally the high-grade stabilization it obtains may avoid the up until now need of a second posterior operation for the necessary additional stabilization with pedicular screws.

## Description

### Terms used

bone-bridge, interconnection, slotted plate, telescopic cylinder, complementary graft.

### TECHNICAL FIELD

Anterior spine-fusion system which consists of telescopic cylinder and osteosynthesis plate that is fixed on the cylinder with screws. The telescopic cylinder fills the gap that can be observed after the removal of broken vertebral bodies and allows the spine to return to its previous height. The plate is held on the upper and lower vertebrae and is attached on the cylinder with screws, thus, enhancing the bridging of the gap. The cylinder-plate-upper and lower healthy vertebral bodies' system, becomes one solid vertebral unit, strong enough to resist turning, bending, axial, and splitting shear forces. The filling up of the cylinder with bone autograft or some other type of graft and the firm contact of the spinal prosthesis, ensures the best qualifications for obtaining bone-bridge and permanent spine-fusion, which is the main goal of this type of operations.

### DESCRIPTION OF PREVIOUS TECHNIQUE.

Nowadays, perforated cylinders of various widths and heights made of titanium alloy are used for the accomplishment of spinal fusion. The cylinder before being put into the spinal gap, is filled with bone autograft or allograft. In this way the gap which remains after the removal of the broken vertebrae is filled. The placement of the cylinder requires distraction of the gap so that the spine will return to its normal height. This is accomplished with the use of a distractor that attaches to the screws that have been screwed on the healthy upper and lower vertebral bodies. The plate or rod which is placed as a second construction bridge between the upper and lower vertebral bodies, compresses the put in between cylinder but without being fixed on it, this allows different movements due to straining forces, resulting in an increased risk of loosening or even migration from the implantation site, which as a result reduces the success of the fusion.

Later on, other unfolding cylinders were developed, but no way of interconnection between the cylinder and the plate was anticipated, resulting in the risk of movement and loosening. Another drawback is that these cylinders aren't provided with an opening for further filling with supplementary graft which is required to be fused after expansion of the telescopic cylinder, thus, leaving a gap equal in size to the length of the distraction which was required in order for spinal height to return to its normal. However, this leaves gaps in the graft inside the cylinder, thus, reducing the chances of success of the secondary spinal-fusion which is also the desired outcome of the operation.

### ADVANTAGES OF THE INVENTION

1. Our prosthesis succeeds in distracting and reducing the Human Spine back to its normal height though expansion of the telescopic expandable cylinder, which consists of two parts 8, 9 and is equipped with an opening (port) 1 allowing complementary graft filling equal in size to the gap which is created after the development of the cylinder. Thus, the continuity and cohesion of the bone graft between the upper and lower healthy vertebrae, and the existence of the optimum conditions for success of the bone-bridge is enhanced. That is permanent spine fusion. The existing systems do not have such ports to fulfill the demand for putting extra and additional graft after its development.
2. The secure fixation of the expanded-developed telescopic cylinder to the overlapping plate by two screws 6, and its fixation to the upper and lower healthy vertebrae with two spongiosa screws 7, interconnects, 'ties' the cylinder, the plate, and the adjacent lower and upper vertebrae, thus creating a solid unit which acts and behaves like one system, eliminating movement and migration risk considerably, while obtaining the best conditions for secondary fusion. Generally speaking, there are no other prosthesis which accomplish both advantages, that is inter-connection with the plate and the port for further filling up with graft.
3. It is easy to implant and the tools required are one screwdriver, and one distractor for unfolding the cylinder.
4. The implantation technique and procedure of the application of this spinal prosthesis accomplishes the goals of such surgical procedures: Viz, a) reduction of the height, b) immobilization and secure fixation of the prosthesis, c) cohesion of the bone-graft it contains. These three goals are pursued by the spine-surgeon in order for him to succeed in immobilizing and securing the prosthesis and obtain permanent secondary fusion.

### REVELATION

Our prosthesis is a combination of one telescopic cylinder and of one plate, which are interconnected.

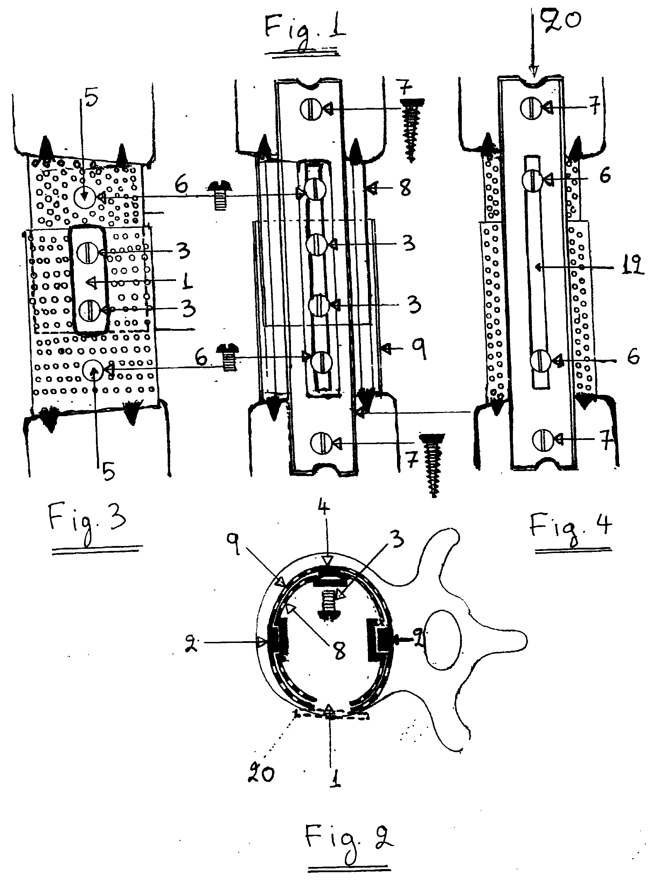

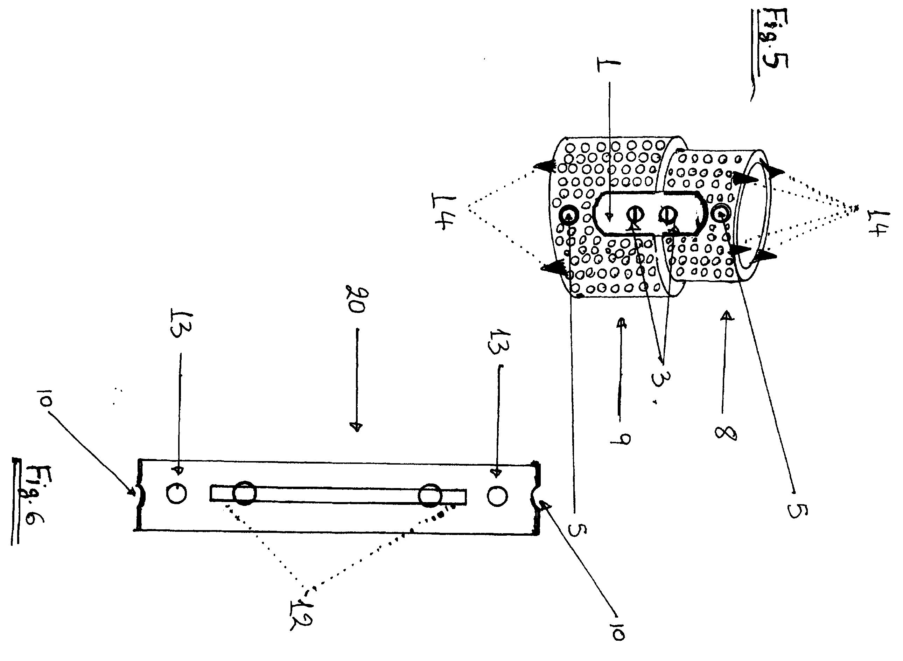

(Fig.5) represents the front of the telescopic cylinder which consists of two parts, the small one 8 which moves into the bigger one 9 and can be stabilized at any position by the screw 3 system. It bears teeth 14 on its sides, of 2,5mm height with in-between footings 3mm wide that prevent further penetration into the vertebrae which causes loss of height. The plate's front 20 can be seen on fig.6 while opening slot 12 is also visible, which after it is attached on the cylinder allows passage of the screws 6 on the threaded holes 5, (fig.3, fig.4, fig.5). It is the screws 6 that hold the placed cylinder securely fixed to the plate 20.

(Fig.3) shows the front side of the cylinder fixed in the chosen position but before placement of the plate 20, (fig.1) its vertical incision, (fig.4) the view of the whole placed prosthesis (with the plate) and (fig.2) the horizontal incision.
The cylinder (fig.5) bears an opening 1 on the surgeon's entry side of access (fig.2 & fig.3) wide enough to allow the passage of two screws 3 that attach to the opposite nerve 4 and secure both 8&9 parts in any desired position. The elongation of the opening port 1 is proportional to the development and increase of the length of the cylinder, allowing easy filling with additional bone graft.

It is a telescopically developing cylinder made of Titanium alloy (fig.5) which consists of two parts, a big one 9 and a smaller one 8. Three radiated nerves 2&4 (fig.2) are used as guides for the elongation of the cylinder while increasing its endurance, the first stabilization screws 3 can be screwed on nerve 4 which is opposite to opening 1, of the moving parts 8&9. The slotted plate 20 (fig.6) that is of the same composition, is equipped with port-slot 12 and non threaded holes 13 on its ends that allow the spongiosa screws 7 that stabilize the plate with the upper and lower vertebral body to be screwed. Screws 6 pass through slot 12 (fig. 1, fig.3 & fig.4) and are screwed in the cylinder's threaded holes 5, thus, securing the cylinder (as a second safety) on the particular development position and fixing it on the plate at any desired position.

Screwing of screws 6 succeeds in two things a) securely connects the cylinder and the plate and b) serves as a second safety for holding the cylinder in the reduction position we have chosen while increasing its endurance at the same time.

The plate bears a notch 10 at its ends so that one side of a compression tool can be attached to it while its other side will be held on the close edge of the opening 1. After completion of this procedure, screw 6 which passes through the slot 12 of the plate 20 is secured in the place of controlled compression on the cylinder's-vertebrae touch points and reinforces its stability, while it 'ties' the upper and lower vertebrae in maximum resistance position to the forces that can act and loosen the stability of the system, or can move the prosthesis.

### EXAMPLE OF THE INVENTION'S APPLICATION

The severity of a human spine's trauma is the result of the injuries of the spine's bones and neural tissue which is enclosed by it. In such a trauma for example fracture of the vertebral body on the L2, sinking and intrusion of bone parts into the spinal canal will result in the risk of neural tissues' injury. The spine becomes unstable and forms an angle at the fracture's point with disastrous consequences. Such a fracture requires surgical confrontation at once. Removal of the mined vertebral body and bone parts that may irritate neural tissues is demanded, and correction of the human spine's angle through replacement of the fractured vertebra by the prosthesis and its fixation to the healthy vertebrae in order to ensure stability and permanent autologous bone-bridging is required.

### Surgery's Description

We place the patient on the operating table on a right lateral position, we approach trans or retro-peritoneally the lumbar spine. We apply the same technique for thoracic spine. We prepare the broken vertebrae as well as the adjacent healthy ones and we remove the broken vertebral body along with the disks in order to completely decompress the spinal canal. The proportionate in size telescopic cylinder 8,9 (fig.5) is placed in-between the gap after we have filled it with bone autograft having port 1 for graft-filling on the surgeon's access point. We unfold the telescopic cylinder at the desired height securing two screws 3 at each final position (fig. 3) and we fill the created gap with complementary graft through port 1. We then place the proportionate in size slotted plate 20 (fig.6) along the spinal column so that it extends from the upper to the lower vertebral body in such a way, that the slot 12 allows the two threaded holes 5 where the lower cylinder's screws 6 are screwed (fig.3) to be seen. The plate is tied on the healthy spines with two screws 7 through the holes 13 (fig.6). Before the screws 6 are screwed, compression of the cylinder-vertebral body's contact surface is done with a compressor which attaches one of its legs on the notch 10 of the plate (fig.4, fig.6), while the other leg is attached to the port's 1 close opening side through the plate's slot. We repeat the same procedure on the other side of the cylinder which contacts to the other vertebrae.

Hence, the slotted plate is tied to the lower cylinder with two screws 6 which are screwed on the threaded holes 5 (fig.3, 4) of the cylinder. The interconnection of all these parts, that is the cylinder, the plate, and the vertebral bodies, succeeds in the high resistance of the system to axial-bending - and shearing forces because all the system acts as one solid unit.

## Claims

1. Spinal prosthesis made of Titanium alloy, which consists of telescopic expandable cylinder consisting of parts 8, 9 and one slotted plate 20, that are interconnected with two screws 6, this prosthesis is put in between and then stabilized in the gap and empty space which occurs between two vertebral bodies after removal of one or more vertebrae including the discs, to the desired and controlled height, and is secured in position with two screws 3 that pass through opening-port 1 which also allows filling of the cylinder with additional bone graft after its expansion.

2. Spinal prosthesis as described in claim 1, whose implantation fills the gap through distraction which results from the cylinder's development, thus, distracting the Spine to the desired height. It is also secured in the final position with a double mechanism of screws 3-6, one on the nerve opposite to the opening 1 which is on the surgeon's entry side, and the second one which fixes the plate 20 onto the cylinder, while it is equipped with port 1 for filling with graft, which (port), gets larger when the cylinder is developed allowing an easy and safe adding of additional amount of graft that may be required. Finally, it interconnects the cylinder-plate and upper and lower vertebral bodies creating a system of the same resistance and behavior to the forces of load that tend to move and loosen the prosthesis.

3. Claim's 1 cylinder bears on its ends and from the surgeon's approach point threaded holes 5 where the two screws 6 that pass through the plate's slot 12 are screwed in order to secure the plate 20 into the compression place on the contact point with the vertebral bodies. They are also the two out of four screws in total that secure the cylinder in the place that we have chosen.

4. Claim's 1 plate (fig.6) comes in different lengths, so that after corporectomy takes place, the cylinder's expansion covers the space between the healthy vertebrae. It bears two holes 13 on its two ends, through which the spongiosa screws 7 are screwed on the upper and lower vertebral bodies. Thus, the plate bridges the healthy vertebral bodies.

5. Claim's 1 plate 20 bears a notch 10 for holding the distraction tool on its ends, in order to manage compression of the cylinder's-body touching point. Its other side attaches on the close side of opening 1.

6. Claim's 1 cylinders bear teeth on their edges, of 2,5mm height with an in-between footing of a 3mm width that stops sinking and loss of height due to intrusion in the upper and lower vertebral bodies. They also have a stabilizing effect against shearing forces that tend to move the prosthesis.

7. Claim's 1 telescopic cylinder, consists of one cylinder of small diameter 8, which moves inside the bigger one 9, sliding along three internal nerve guides 2-4. At the nerve 4 which is opposite to the port 1, we pass and screw two screws 3 which stabilize the cylinder at any length providing also compression to the adjacent healthy vertebrae.

8. Claim's 1 telescopic cylinder, can be round or oval and its two edges may be of trapezium or not shape, in order to fit with the anatomic needs that normally depend on the level of the spine we operate.

9. Claim' s 1 telescopic cylinder must be available at such external diameters so that it will fit the different diameters of every vertebra, from the small cervical ones to the bigger lumbar ones.

10. Claim's 1 telescopic cylinder for replacement of small cervical vertebrae, has its port 1 at its front part along with its threaded holes 5, for screwing plate 20 with the two screws 6 through port 12.
